# EUROPÄISCHE PATENTSCHRIFT

(11) **EP 1 471 951 B1**
(45) Veröffentlichungstag und Bekanntmachung des Hinweises auf die Patenterteilung: **11.03.2009**
(21) Anmeldenummer: 03734723.4
(22) Anmeldetag: 31.01.2003
(51) Int. Cl.: A61L 15/42, A61L 24/00, A61L 15/24, A61L 24/04

(54) **BLUTSTILLUNGSMITTEL ENTHALTEND POLYVINYLALKOHOL UND SEINE BEREITSTELLUNG FÜR DIE MEDIZIN**
HAEMOSTATIC AGENT CONTAINING POLYVINYL ALCOHOL AND PROVISION OF THE SAME FOR MEDICAL USE
AGENT HEMOSTATIQUE CONTENANT DE L'ALCOOL POLYVINYLIQUE ET SA MISE A DISPOSITION POUR LA MEDECINE

(30) Priorität: 31.01.2002 DE 10204819
(43) Veröffentlichungstag der Anmeldung: 03.11.2004
(73) Patentinhaber: Aesculap AG, 78532 Tuttlingen (DE)
(72) Erfinder: FRIEDRICH, Volker, 78579 Neuhausen (DE); ODERMATT, Erich, K., CH-8200 Schaffhaussen (CH); WEIS, Christine, 78532 Tuttlingen (DE)
(74) Vertreter: Patentanwälte Ruff, Wilhelm, Beier, Dauster & Partner
(86) Internationale Anmeldenummer: PCT/EP2003/000991
(87) Internationale Veröffentlichungsnummer: WO 2003/063922

(56) Entgegenhaltungen:
- EP-A- 0 693 290
- WO-A-00/27327
- WO-A-01/82896
- WO-A-02/09789
- WO-A-02/22059
- WO-A-98/03203
- WO-A-03/000155
- DATABASE WPI Section Ch, Week 200067 Derwent Publications Ltd., London, GB; Class A96, AN 2000-682020 XP002239815 & JP 2000 249700 A (TOA IYO DENSHI KK), 14. September 2000 (2000-09-14)
- DATABASE WPI Section Ch, Week 198707 Derwent Publications Ltd., London, GB; Class A96, AN 1987-046613 XP002239816 & JP 62 004232 A (UNITIKA LTD), 10. Januar 1987 (1987-01-10)
- DATABASE WPI Section Ch, Week 199544 Derwent Publications Ltd., London, GB; Class A96, AN 1995-342809 XP002239817 & RU 2 031 661 C (EKOMEDSERVIS RES PRODN ENTERP), 27. März 1995 (1995-03-27)
- DATABASE WPI Section Ch, Week 199339 Derwent Publications Ltd., London, GB; Class A14, AN 1993-311051 XP002239818 "Haemostatic material prepn. - by dissolving gelatin in soft water contg. auxiliary agent, heating, and then adding polyvinyl alcohol, etc." & TW 206 919 A (SONG J), 1. Juni 1993 (1993-06-01)
- DATABASE WPI Section Ch, Week 199947 Derwent Publications Ltd., London, GB; Class A14, AN 1999-551834 XP002239819 & CN 1 223 316 A (ZHOU Y), 21. Juli 1999 (1999-07-21)
- DATABASE WPI Section Ch, Week 199301 Derwent Publications Ltd., London, GB; Class A14, AN 1993-007159 XP002239820 & SU 1 705 304 A (SOROKIN A YA), 15. Januar 1992 (1992-01-15)
- DATABASE WPI Section Ch, Week 197919 Derwent Publications Ltd., London, GB; Class A96, AN 1979-36282B XP002239821 & JP 54 042880 A (SEISAN KAIHATSU KAGAKU KENKYUS), 5. April 1979 (1979-04-05)
- DATABASE WPI Section Ch, Week 198843 Derwent Publications Ltd., London, GB; Class A18, AN 1988-302571 XP002239822 & JP 63 220876 A (NIPPON SODA CO), 14. September 1988 (1988-09-14)

## Beschreibung

Die vorliegende Erfindung betrifft ein Blutstillungsmittel und seine Bereitstellung zur Anwendung in der Medizin.

Bei Menschen und Tieren kann es aufgrund von Verletzungen, medizinischer Behandlung oder chirurgischen Eingriffen zu Blutaustritt an der betroffenen Stelle auf der Körperoberfläche oder im Körperinneren kommen. Nach einer Phase des Blutaustritts setzt eine natürliche Blutgerinnung ein. Die Blutgerinnung bezeichnet das Erstarren des flüssigen Blutes als physiologischer Schutzmechanismus. Die Blutgerinnung ist ein komplexer in mehreren Phasen ablaufender Vorgang, der zur Bildung von unlöslichem Fibrin aus dem im Blutplasma vorhanden Fibrinogen führt. An dem Vorgang sind viele Faktoren beteiligt, die am Zustandekommen des Gerinnsels zusammenwirken.

Die natürliche Blutgerinnung ist jedoch ein langsamer Prozess, der erst mit gewisser Verzögerung nach Eintritt des Traumas einsetzt und bis zur Blutstillung einige Zeit dauert, während indessen weiterer Verlust von Blut auftritt. Es wurden deshalb Mittel zur Verbesserung der Blutstillung vorgeschlagen. Im einfachsten Fall durch Wundabdeckung und Druckverband. Eine andere Möglichkeit ist, blutgerinnungsaktive Substanzen, wie Fibrinkleber, in den Wundbereich einzubringen. In jüngerer Zeit wurden auch bioverträgliche partikelförmige Substanzen auf Basis von Keramik, Biopolymeren wie Dextran oder synthetischen Polymeren entwickelt, die in den Wundbereich eingebracht eine hämostatische Wirkung ausüben. Als Beispiel hierzu ist das US-Patent Nr. 6,060,461 zu nennen.

Blutstillungsmittel enthaltend PVA oder Mischpolymere davon, auch in Pulverform, werden in den Patentdokumenten JP2000249700, RU2031661 und SU1705304 offenbart.

Bei den bekannten Mitteln zur Hämostase kann jedoch ihre Wasserlöslichkeit dazu führen, dass sie besonders bei starken Blutungen schnell aus dem Wundgebiet weggeschwemmt werden, und deshalb ihre Wirkung nicht voll erfüllen können. Bei zur Blutungshemmung eingesetzten Feststoffen kann es durch mangelnde Abbaubarkeit in physiologischem Milieu zu Problemen kommen. Auch können mangelnde Biokompatibilität von Begleitstoffen oder Abbauprodukten der bekannten Mittel zur Blutstillung nachteilige Wirkung ausüben. Ferner stellt sich bei partikulären Blutstillungsmitteln das Problem, dass sie für ausgedehnte Wundgebiete umständlich anzuwenden sind.

Es stellt sich daher die Aufgabe, ein biokompatibles, schnell wirksames Blutstillungsmittel zur Verfügung zu stellen, das die Probleme mit Produkten aus dem Stand der Technik überwindet, einfach und kostengünstig herzustellen ist und in der medizinischen Praxis mit üblichen Methoden leicht und zuverlässig anzuwenden ist.

Die Aufgabe wird gelöst durch ein Blutstillungsmittel umfassend mindestens einen porösen Polyvinylalkohol (PVA) in festem Zustand ausgewählt aus unvernetztem PVA mit einem Molekulargewicht von 15000 bis 400000, vernetztem PVA und Mischungen davon, wobei der poröse PVA in Form eines Pulvers mit einer Teilchengröße von 100 nm bis 1 mm vorliegt und eine Porengröße von 10 nm bis 0,05 mm aufweist.

PVA ist ein biokompatibles synthetisches Polymer, das wegen seiner ausgezeichneten Bioverträglichkeit (Biokompatibilität) zum Einsatz in Wundbereichen des Körpers von Mensch oder Tier hervorragend geeignet ist. Beispielsweise wird PVA in vivo nicht modifiziert, es führt zu keinen entzündlichen Reaktionen und es wird nur in geringem Umfang in Körperorganen akkumuliert. Prinzipiell ist PVA ein biologisch abbaubares synthetisches Polymer mit einem Kohlenstoff-Kohlenstoff-Rückgrat. Es ist ein enzymatischer Abbaumechanismus bekannt, der mehrere Tage dauert. Die Hydroxylgruppe wird dabei zu einer Ketogruppe oxidiert und unter Spaltung der Kohlenstoff-Kohlenstoff-Bindung in Methylketone und Carboxylsäuren hydrolysiert.

Die Ausscheidung des PVA ohne Degradation erfolgt größtenteils über die Niere, wobei die Ausscheidungsgeschwindigkeit vom Molekulargewicht abhängig ist. PVA mit einem Molekulargewicht von weniger als 15000 g/mol wird innerhalb weniger Stunden aus dem Körper ausgeschieden, was für die vorgesehene Anwendung in der Blutstillung (Hämostase) zu schnell erfolgt.

Mit besonderem Vorteil kann der poröse PVA als Blutstillungsmittel in Form eines PVA-Schaums vorliegen. Bei der Erfindung liegt der poröse PVA als Blutstillungsmittel in Form eines Pulvers vor.

Das erfindungsgemäße Blutstillungsmittel kann sich dadurch auszeichnen, dass der PVA-Schaum offenporig ist. Der erfindungsgemäße PVA-Schaum ist mit Vorteil ein Strukturschaum mit einem offenen Gerüst, der ein Eindringen von flüssigem Medium ermöglicht.

Beim erfindungsgemäßen PVA-Schaum zur Blutstillung ist die disperse Phase des Schaums Luft. Von dieser Gasphase ist keine nachteilige Wirkung auf den Patienten zu erwarten. Als Treibmittel für den PVA-Schaum kann bevorzugt Kohlendioxid verwendet sein.

Es ist im Fachbereich bekannt, PVA-Schaum in der Medizin einzusetzen. Als Beispiele sind zu nennen in der Vacuum Assisted Closure (VAC) in der Traumatologie, zur Defektkonditionierung in der Tumorchirurgie der Haut und bei chronischen Ulcera.

Ein entsprechend der Größe und Beschafffenheit der Wundfläche und der Blutungsintensität ausgewähltes PVA-Schaumprodukt wird in den Wundbereich eingesetzt. Für unregelmäßige und tiefe Wunden sind PVA-Schaumpartikel vorteilhaft.

Bei Kontakt mit flüssigem Blut quillt der Schaum auf und kleidet auf diese Weise die Wunde aus. Die überschüssige Feuchtigkeit wird aufgenommen und von der Wunde weggeleitet. Der erfindungsgemäße PVA-Schaum kann dabei ähnlich einem Molekularsieb wirken. Der erfindungsgemäße PVA-Schaum kann sich vorteilhaft durch eine hohe Gesamtaufnahmekapazität für Flüssigkeit auszeichnen. Bevorzugt kann das Blutstillungsmittel in trockenem Zustand ein Flüssigkeitsaufnahmevermögen vom 15- bis 55fachen des Produkteigengewichts, insbesondere dem 15- bis 30fachen des Produkteigengewichts aufweisen. Ferner kann sich der erfindungsgemäße PVA-Schaum durch ein gutes Retentionsvermögen auszeichnen. Bevorzugt kann das Blutstillungsmittel ein Flüssigkeitsretentionsvermögen vom 15- bis 45fachen des Produkteigengewichts, insbesondere 15- bis 25fachen des Produkteigengewichts aufweisen. Neben der hohen Gesamtaufnahmekapazität und dem Retentionsvermögen ist auch die Anwendung des PVA-Schaum in Körperbereichen ermöglicht, die Druck ausgesetzt sind, ohne dass Flüssigkeitsaustritt erfolgt.

Durch die Aufnahme der Flüssigkeit wird eine hohe Konzentration von roten Blutkörperchen um den PVA-Schaum gebildet. Bei PVA-Teilen kann diese Anlagerung um die Teile erfolgen. Durch Aufnahme der im Blut enthaltenen Flüssigkeit ins Innere des PVA-Schaums wird das Blut sozusagen dehydriert. Die Blutflüssigkeit wird vom PVA-Schaum absorbiert, während größere Blutbestandteile wie Blutzellen, Blutplättchen, Thrombin, Fibrinogen, Proteine wie Albumin an der Oberfläche des PVA-Schaums haften. Durch Quellung des PVA-Schaums werden die Zellen weiter konzentriert. Auf diese Weise wird die normale Blutgerinnungskaskade beschleunigt.

Auf diese Weise kann der Blutaustritt gestoppt und damit der Blutverlust begrenzt werden. Das koagulierte Blut bildet einen gewissen natürlichen Wundverschluss. Es kann somit das Eindringen von Fremdmaterial wie beispielsweise Mikroorganismen, chemischen Substanzen oder Partikeln in den Körper des Patienten vermeiden. Bei Anwendung des Blutstillungsmittels im Körperinneren kann neben schleichendem Blutverlust auch die Ausbreitung von biologischen Substanzen und Mikroorganismen und damit das Infektionsrisiko begrenzt werden.

Das erfindungsgemäße Blutstillungsmittel ist zur Anwendung in der Humanmedizin und Veterinärmedizin geeignet. Anwendungsbereiche umfassen beispielsweise Dermatologie mit Wundbehandlung bei akuten Traumazuständen und chronischen Hautläsionen, Chirurgie einschließlich Weichteilchirurgie, Gefäßchirurgie, plastische Chirurgie und Orthopädie, Zahnheilkunde und Notfallversorgung. Aus Tierversuchen ist bekannt, dass Körperzellen sehr gut in lyophilisierte, trockene, poröse PVA-Membranen einwachsen können.

Die Aufnahmefähigkeit des PVA-Schaum für Flüssigkeit, sozusagen seine Saugfähigkeit wird insbesondere durch die Porosität bestimmt. Mit Vorteil kann sich das erfindungsgemäße PVA-Schaumprodukt zur Blutstillung dadurch auszeichnen, dass die Porengröße im Schaum 1 nm bis 5 mm, insbesondere 10 nm bis 0,5 mm beträgt. Bei einem Pulver eine Porengröße von 10 nm bis 0,05 mm bevorzugt. Die Porosität kann durch die Prozessbedingungen bei Herstellung und Verarbeitung des PVA eingestellt werden. Durch geeignete Wahl der Ausgangsmaterialien, der Verfahrensweise und Verfahrensbedingungen ist eine maßgeschneiderte Anpassung des Aufnahmeverhaltens des erfindungsgemäßen PVA-Schaums an die praktischen Anforderungen möglich. Das Resorptionsverhalten in vivo des PVA-Materials kann durch geeignete Wahl des Vernetzungsgrads des PVA beeinflusst werden. Außerdem kann die Porosität über die Vernetzung beeinflusst werden.

In einer Ausführungsform der Erfindung kann die Herstellung des PVA-Schaums über Gefriertrocknung von aufgeschäumten PVA-Lösungen erfolgen. In einer anderen Ausführungsform der Erfindung kann die Herstellung des PVA-Schaums über Gefriertrocknung von physikalisch vernetzten Hydrogelen erfolgen. In einer weiteren Ausführungsform der Erfindung kann die Herstellung des PVA-Schaums über Gefriertrocknung von chemischen vernetzten Hydrogelen erfolgen. In noch einer anderen Ausführungsform der Erfindung kann die Herstellung des PVA-Schaums über Gefriertrocknung von physikalisch vernetzten Partikeln erfolgen. In noch einer weiteren Ausführungsform der Erfindung kann die Herstellung des PVA-Schaums über Gefriertrocknung von chemisch vernetzten Partikeln erfolgen.

Mit Vorteil kann der PVA-Schaum als Blutstillungsmittel einen Luftanteil nach Gefriertrocknung von 60 bis 90 % aufweisen. In einer Variante kann der PVA-Schaum als Blutstillungsmittel einen Luftanteil nach Kompaktieren von 10 % aufweisen.

Zur Verwendung als Blutstillungsmittel kann der erfindungsgemäße PVA-Schaum schneidbar, insbesondere elastisch sein.

In einer besonderen Ausführungsform kann sich das erfindungsgemäße Blutstillungsmittel dadurch auszeichnen, dass mindestens eine Wirksubstanz, insbesondere ein Medikament zugesetzt ist. In einer bevorzugten Ausführungsform kann dem Blutstillungsmittel gemäß der Erfindung mindestens eine die Blutgerinnung fördernde Substanz zugesetzt sein. Als Beispiele weiterer Substanzen, die dem Blutstillungsmittel zugesetzt sein können, sind Antibiotika, Entzündungshemmer, Wachstumsförderer oder Koagulationsbeschleuniger wie Thrombin, Fibrinogen und Aprotinin sowie auch Calciumchlorid (CaCl₂) zu nennen. Mit Vorteil können antibakterielle, bakteriostatische und entzündungshemmende Stoffe dem erfindungsgemäßen Blutstillungsmittel zugesetzt sein, wie beispielsweise Propolis. Auf diese Weise kann das Blutstillungsmittel in infiziertem Gewebe eingesetzt werden, um die Wundheilung zu fördern.

In einer anderen Ausführungsform kann sich ein erfindungsgemäßes Blutstillungsmittel dadurch auszeichnen, dass dem PVA mindestens ein höhermolekulares Biopolymer aus der Gruppe der Proteine beigemischt ist, dessen Sekundärstruktur zur Stabilisierung des 3-dimensionalen Netzwerkes genutzt wird. Insbesondere kann mit Erhöhung der Temperatur und/oder mit chemischen Substanzen die Sekundär- und Tertiärstruktur der Proteine teilweise oder ganz entfaltet werden und in der Mischung mit PVA nach ganzer oder teilweiser Rückstellung der vorherigen Bedingungen eine physikalische Vernetzung und Stabilisierung dieses PVA-Proteinnetzwerkes erfolgen. Ein solches Protein-Netzwerk kann als extrazelluläres Scaffold für Zellen des Bindegewebes Verwendung finden.

Mit besonderem Vorteil kann der erfindungsgemäß eingesetzte PVA ein Molekulargewicht von 50000 bis 300000 g/mol aufweisen. Bei höherem Molekulargewicht ist insbesondere ein mit dem PVA hergestelltes Schaumprodukt stabiler. Bevorzugt kann für ein PVA-Schaumprodukt ein PVA mit Molekulargewicht von 150000 bis 250000 verwendet werden.

In einer Ausführungsform kann der PVA aus einem Gemisch von niedermolekularen und hochmolekularen Komponenten gebildet sein, wobei insbesondere die hochmolekulare Komponente PVA ist.

Polyvinylalkohol ist wegen seiner Hydroxylgruppen wasserlöslich. Durch eine Modifizierung können die Löslichkeit und andere chemische und physikalische Eigenschaften des Polymers verändert werden. Die Modifizierung von PVA kann durch chemische Modifizierung, durch physikalische Modifizierung oder eine Kombination von Modifizierungen vorgenommen sein. Als Beispiele für chemische Modifizierung sind Copolymerisation, Pfropfung oder chemische Vernetzung zu nennen. Als Beispiel für physikalische Modifizierung sind physikalische Vernetzung, Ausbildung orientierter Molekularstrukturen, Hydrogele und Kristallitbildung zu nennen.

Eine chemische Vernetzung des rohen PVA kann allgemein über die alkoholischen Gruppen in einer Additionsreaktion mit Diisocyanat oder in einer Kondensationsreaktion mit einer mehrfunktionellen Säure durchgeführt werden. Eine reversible Unlöslichkeit durch Vernetzung ist zur biologischen Ausscheidung des PVA bevorzugt. Von besonderer Bedeutung für die Bereitstellung des erfindungsgemäßen Produkts für die Medizin ist, dass unter den physiologischen Bedingungen im Körper des Patienten keine toxischen oder gesundheitsschädlichen Substanzen entstehen. Aus diesem Grund ist eine hydrolysierbare Vernetzung, insbesondere mittels mehrwertiger Carbonsäuren, beispielsweise in Form von Anhydriden, erfindungsgemäß bevorzugt. Es kann auch eine enzymatisch spaltbare Vernetzung verwendet werden. Die Vernetzung kann mit einem metabolisierbaren Diisocyanat vorgenommen sein, das eine spaltbare Bindung trägt, wie beispielsweise eine Esterbindung. So können keine Urethanbrücken entstehen, die nicht oder erst nach langer Zeit im Körper abgebaut werden.

PVA mit einem niederen Molekulargewicht von 15000 bis 40000 g/mol ist in vernetzter Form geeignet. In einer Ausführungsform der Erfindung kann PVA mit einem Molekulargewicht von 15000 bis 400000 g/mol chemisch vernetzt sein. Mit Vorteil kann sich das erfindungsgemäße Produkt dadurch auszeichnen, dass die Vernetzung mittels Vernetzern vorgenommen ist, die eine in vivo reversible Vernetzung, insbesondere durch chemische Hydrolyse reversible Vernetzung ergeben. Bei resorbierbaren Polymeren sind die Vernetzungsstellen vorzugsweise chemisch hydrolysierbar, nicht enzymatisch spaltbar. In einer bevorzugten Ausführungsform kann beim erfindungsgemäßen Produkt zur Blutstillung die chemische Vernetzung durch Veresterung vorgenommen sein.

Erfindungsgemäß bevorzugt können als Vernetzer mehrwertige Dicarbonsäuren und/oder ihre Derivate vorgesehen sein. Die Veresterung der alkoholischen Gruppen im PVA mit Dicarbonsäuren zeichnet sich als reversible Vernetzungsreaktion aus. Als Vernetzungsmittel können insbesondere Anhydride von Carbonsäuren verwendet sein. Als Beispiele solcher Vernetzungsmittel sind Bernsteinsäureanhydrid oder Oxalsäureanhydrid zu nennen. Bernsteinsäureanhydrid ist reaktiver als die Bernsteinsäure, da Ringöffnungsenergie frei wird.

Auch andere Vernetzer mit mindestens zwei funktionellen Gruppen sind möglich. Ferner können als Vernetzungsmittel Verbindungen mit Doppelbindungen eingesetzt werden. Als Beispiele hierfür sind Imide und Acrylate zu nennen. Desweiteren können auch hochmolekulare Vernetzungsmittel, z. B. Dextranaldehyd verwendet werden. In Weiterbildung kann sich ein erfindungsgemäßes Blutstillungsmittel dadurch auszeichnen, dass als Vernetzer ein hochmolekularer Dextranaldehyd und/oder ein Derivat davon verwendet ist.

Wenn die Vernetzungsreaktion, in Lösung durchgeführt wird, ist ein kurzkettigeres Vernetzungsagens vorteilhafter, da die Wahrscheinlichkeit einer intramolekularen Reaktion mit nur einer Polymerkette gering ist. Auf diese Weise sind Oxalsäure und ihre Derivate wegen der kürzeren Molekülkette erfindungsgemäß bevorzugt.

In einer anderen Ausführungsform der Erfindung kann PVA mit einem Molekulargewicht von 15000 bis 400000 g/mol physikalisch vernetzt sein. Mit Vorteil kann die physikalische Vernetzung durch Kristallitbildung vorgenommen sein. Bei einer solchen physikalischen Vernetzung ist ein dreidimensionales Netzwerk von PVA-Molekülen ausgebildet, das durch Kristallite als physikalische Vernetzungspunkte zusammengehalten wird.

Eine weitere physikalische Vernetzungsart beinhaltet die Mischung mit Biopolymeren und die Nutzung der Sekundärstruktur α-Helix, β-Faltblatt und die Triplehelix von Proteinen. Die Auffaltung der Sekundärstruktur kann mittels Temperatur und/oder chemischen Agenzien, wie Harnstoff, erfolgen. Die Rückfaltung der Biopolymerstruktur in der Mischung mit PVA erfolgt durch Verdünnung oder Entfernung des chemischen Agens bzw. durch Temperaturerniedrigung. Dabei kommt es zu einer physikalischen Vernetzung zwischen den Polymeren PVA und dem Biopolymer, welche biologisch abbaubar sind. Als mögliche Biopolymere sind beispielsweise Proteine wie Keratine, Kollagene, Fibronektin sowie Kohlenhydrate wie Zuckerpolymere und Chitosan zu nennen.

Zur Ausbildung einer physikalischen Vernetzung kann eine wässrige Lösung von PVA für 6 bis 48 Stunden bei -20°C eingefroren und dann bei 25°C über 2 bis 6 Stunden aufgetaut werden, so dass sich die gewünschte Vernetzung ausbildet. Gemäß der Erfindung kann die Darstellung von PVA-Hydrogelen über einen Gefrier-/Tauzyklus vorgenommen werden, der mit Vorteil insbesondere mehrmals wiederholt werden kann. Bei der physikalischen Vernetzung durch Gefrier-/Tauzyklen können bei unterschiedlichen Temperaturen verschiedene Phasen zugeordnet sein.

Die PVA-Ketten können auch modifiziert sein, indem nur wenige Hydroxylgruppen des PVA, vorzugsweise über Ester- und/oder Ethergruppen mit zusätzlichen Resten verbunden sind. Für eine solche Modifikation eignen sich insbesondere Fettsäuren und/oder Alkohole mit einer Kettenlänge von C2 bis C16. Es können auch Aminosäuren oder Peptide verknüpft werden. Es reichen 1 bis 10, insbesondere 1 bis 2 Reste pro Molekül PVA aus. Durch eine solche Modifikation kann eine verbesserte Gelbildung unter physiologischen Bedingungen erreicht werden.

In einer anderen Ausführungsform kann PVA in Mischung mit einer hochmolekularen Komponente vorliegen, die kein PVA ist. Eine solche hochmolekulare Komponente kann in einer Menge von 0,5 bis 4 Gew.-%, insbesondere 1 bis 2 Gew.-% vorliegen. Ferner kann die nicht aus PVA gebildete hochmolekulare Komponente zur zusätzlichen Medikamentenaufnahme vorgesehen sein.

Mit Vorteil kann dem erfindungsgemäßen PVA als hochmolekulare Komponente ein Zuckerpolymer zugesetzt sein. Insbesondere kann das Zuckerpolymer ausgewählt sein aus der Gruppe bestehend aus Carboxymethylcellulose, Dextran, Hydroxymethylcellulose, Hydroxyethylstärke, Chitosan und/oder Stärke.

In Weiterbildung kann sich ein erfindungsgemäßes Blutstillungsmittel dadurch auszeichnen, dass das Zuckerpolymer ausgewählt ist aus der Gruppe der Chitine, insbesondere Chitosan ist.

Bei schwach vernetzten, trockenen Polymeren tritt in wässrigen Medien oder geeigneten Lösemitteln Quellung auf, wobei die Quellung an der Oberfläche beginnt und ins Innere fortschreitet. Die Quellungsgeschwindigkeit wird dabei nicht durch die Diffusionskoeffizienten der Quellungsmittel, sondern durch die Diffusionsgeschwindigkeit der Segmente des Polymeren beeinflusst. Je mehr die Quellung fortschreitet, um so stärker machen sich die elastischen Rückstellkräfte der vernetzten Polymerketten bemerkbar. Gele weisen viskoelastische Eigenschaften auf. Die Quellung erfolgt bis zu einem maximalen Wert. Im Gegensatz zu unvernetzten Polymeren, aus denen wässrige Lösungen in variabler Konzentration hergestellt werden können, werden chemisch vernetzte Polymere nicht gelöst, es sei denn, die Vernetzungsbrücken sind hydrolysierbar.

Kovalent und physikalisch vernetzte Gele unterscheiden sich in der Konzentrations- und der Polymerisationsgradabhängigkeit der Moduln. In beiden Fällen wird der Modul durch die Konzentration an Netzstellen kontrolliert. Prinzipiell kann man sich physikalische Netzwerke wie einen Teller Spaghetti vorstellen, die sich untereinander verhaken. Bei kovalent verknüpften Netzwerken ist dafür nur der Quellungsgrad und somit die Konzentration des Vernetzers im Gel verantwortlich. Der Polymerisationsgrad ist unendlich hoch und kann auch durch Scherung nicht verändert werden. Dies spielt eine große Rolle bei normalen Körperbewegungen, da der Polymerisationsgrad unverändert bleibt. In kovalenten Netzwerken ist kein Abgleiten der Ketten möglich. Bei physikalischen Netzwerken werden unter Scherung die Netzpunkte aufgelöst und wieder verknüpft. Ein nur physikalisch vernetztes Polymer kann unter bestimmten Bedingungen von einer Oberfläche wieder abgewaschen werden und so in der Chirurgie insbesondere für eine geringe Verweilzeit und geringe Funktionsdauer im Körper geeignet sein. Ein besonderer Vorteil kovalent vernetzter Polymere ist, dass ihre Eigenschaften nach Wunsch eingestellt werden können.

Polyvinylalkohol in Form eines Hydrogels ist ein gummiartiges Material, wobei seine Elastizität über definierte Netzpunkte so eingestellt werden kann, dass sie weichem Körpergewebe oder Muskeln sehr nahe kommt. Gleichzeitig weist PVA eine hohe Zugfestigkeit auf.

Gemäß der Erfindung kann das Molekulargewicht des PVA bzw. der Mischung so gewählt sein, dass er im wesentlichen ohne Degradation der PVA-Moleküle über die Niere ausscheidbar ist. Gegebenenfalls kann PVA nach einer Hydrolyse bzw. nach Aufhebung der Vernetzung, im wesentlichen ohne Degradation der PVA-Moleküle über die Niere ausscheidbar sein.

Erfindungsgemäß bevorzugt kann der Vernetzungsgrad des medizintechnischen PVA-Produkts so eingestellt sein, dass seine Funktionsdauer im Operationsgebiet 5 bis 21 Tage, insbesondere 5 bis 14 Tage beträgt. Zur Hämostase ist es wünschenswert, dass das Material mindestens für 5 Tage im Behandlungsgebiet verbleibt. Eine Steuerung der Ausscheidungszeit kann über eine Einstellung von Parametern erfolgen, die die PVA-Ausscheidung beeinflussen. Als Einflussfaktoren sind beispielsweise chemische Vernetzung, physikalische Vernetzung des PVA-Materials, Schichtdicke, Mischungszusammensetzung sowie Zusatz von Additiven wie Zuckerpolymeren zu nennen.

Mit Vorteil kann die makroskopische Auflösung des erfindungsgemäßen medizintechnischen Produkts unter physiologischen Bedingungen, insbesondere in vivo, 7 bis 60 Tage betragen. Die Verweilzeit des PVA im Körper hängt auch ab vom hydrodynamischen Radius der Polymermoleküle. Erfindungsgemäßer PVA kann mit Vorteil einen hydrodynamischen Radius von 5 nm bis 15 nm, insbesondere von 5 nm aufweisen.

Mit besonderem Vorteil kann der vernetzte PVA ein in physiologischer Umgebung vorteilhaftes Benetzungsverhalten aufweisen. Das erfindungsgemäße medizintechnische Produkt zur Blutstillung kann sich besonders dadurch auszeichnen, dass der vernetzte PVA eine Struktur aufweist, die in physiologischem Milieu einen Stoffaustausch von kleinen Molekülen erlaubt.

Zur Stabilisierung des PVA-Schaums können Zusatzstoffe ausgewählt aus der Gruppe der ionischen Tenside, nichtionischen Tenside, Polymere und Mischungen davon zugesetzt sein. Ferner können Geliermittel und/oder Verdickungsmittel zugesetzt sein. Solche Verdickungsmittel können ausgewählt sein aus der Gruppe bestehend aus Cellulose, Methylcellulose, Hydroxypropylcellulose, Hydroxypropylmethylcellulose, Ethylmethylcellulose, Carboxymethylcellulose (CMC), Natriumcarboxymethylcellulose und Mischungen davon.

Durch eine Finish-Behandlung kann eine weitere Anpassung der Eigenschaften des PVA-Schaums gemäß der Erfindung erreicht werden. Als Beispiel ist eine Beschichtung mit Carboxymethylcellulose zu nennen.

Die Erfindung umfasst ferner ein Verfahren zur Herstellung eines PVA-Schaumprodukts. Das Verfahren ist dadurch gekennzeichnet, dass aus mindestens einem PVA ausgewählt aus der Gruppe bestehend aus unvernetztem PVA mit einem Molekulargewicht von 15000 bis 400000 g/mol, vernetztem PVA und Mischungen davon eine Lösung gebildet wird, diese Lösung dann auf eine geeignete Porengröße verschäumt, eingefroren und lyophilisiert wird.

Das Verschäumen der PVA-Lösung kann gemäß dem Fachmann bekannten Verschäumungsverfahren vorgenommen werden. Als Beispiele sind Verschäumung durch Rotormixer, Dampfzufuhr, Druckluftzufuhr, Extrusion mit Treibmittel und Zusatz von Treibmittel zu nennen. Mit besonderem Vorteil kann als Treibmittel Kohlendioxid eingesetzt werden, da mit Kohlendioxid keine nachteiligen Wirkungen beim Patienten zu erwarten sind.

In einer Ausführungsform kann die Herstellung von PVA-Schaum über die Gefriertrocknung aufgeschäumter PVA-Lösungen erfolgen. Dabei werden 1 bis 30 Gew.-% PVA in Wassser gelöst und in einer Verschäumeinrichtung wie einem Mixer aufgeschäumt. Der erhaltene Schaum wird eingefroren und anschließend lyophilisiert. Zur Stabilisierung können ionische oder nichtionische Tenside oder auch Polymere wie PEG (Polyethylenglykol) zugegeben werden. Gegebenenfalls können bioverträgliche Geliermittel oder Verdickungsmittel zugesetzt werden. Als Beispiel sind Cellulose, Methylcellulose, Hydroxypropylcellulose, Hydroxypropylmethylcellulose, Ethylmethylcellulose, CMC, Natriumcarboxymethylcellulose zu nennen, die auch in der Lebensmittelindustrie Verwendung finden.

In einer anderen Ausführungsform kann die Herstellung von PVA-Schaum über die Gefriertrocknung von vernetzten Hydrogelen erfolgen. Hierzu können physikalisch oder chemisch vernetzte PVA-Hydrogele verwendet werden. Zur genauen Steuerung der Porengröße können die Hydrogele lyophilisiert werden. Durch Gefrier-Auftau-Zyklen hergestellte oder chemisch vernetzte PVA-Hydrogele können sich durch relativ homogene Porengröße und Porenverteilung auszeichnen. Die Porengröße beeinflusst insbesondere das Flüssigkeitsaufnahmevermögen des Materials.

In einer weiteren Ausführungsform kann die Herstellung von PVA-Schaum über Gefriertrocknung von vernetzten Partikeln erfolgen. Hierbei können physikalisch oder chemisch vernetzte Partikel verwendet werden. Die Partikelgröße kann im Bereich von Nanometern bis Millimetern liegen. Durch geeignete Wahl der Vernetzungsbedingungen und des Vernetzungsgrades kann die Porosität gesteuert werden. Es können erfindungsgemäß monodisperse Partikel von 100 nm bis 100 µm mit Porengrößen im Bereich von 10 nm bis 1 µm hergestellt werden.

Des weiteren betrifft die Erfindung die Bereitstellung des medizintechnischen Produkts wie es oben beschrieben ist zur Verwendung bei der Blutstillung in der Humanmedizin und Veterinärmedizin. Das erfindungsgemäße Produkt auf Basis von PVA kann je nach den medizinischen Erfordernissen und der gewünschten Einsatzweise in verschiedenen Formen bereitgestellt bzw. zur Verwendung vorbereitet werden. Bevorzugt ist die Bereitstellung in Form eines Schaumprodukts.

Weitere Merkmale und Einzelheiten der Erfindung ergeben sich aus der nachfolgenden Beschreibung von bevorzugten Ausführungsformen in Form von Beispielen. Dabei können die einzelnen Merkmale jeweils für sich alleine oder zu mehreren in Kombination miteinander verwirklicht sein. Die Beispiele dienen lediglich der Erläuterung der vorliegenden Erfindung, die in keiner Weise darauf beschränkt sein soll.

### Beispiel 1

### Bioverträglichkeit

Die Biokompatibilität der untersuchten PVA-Produkte ist ausgezeichnet, z.B. zeigt die akute systemische Toxizitätsprüfung in Mäusen keinerlei Unterschied zu den injizierten KontrollLösungen. Die Injektionen wurden teilweise intravenös und teilweise intraperitoneal durchgeführt. Die Tiere wurden nach 4, 24, 48 und 72 Stunden nach der Injektion beobachtet. Dasselbe gilt z.B. auch für die Cytotoxizitätsprüfungen mit Mausfibroblasten, welche ebenfalls gute Ergebnisse zeigten. Es wurde keinerlei Veränderung der Zellmorphologie oder Hinweise toxischer Wirkung gefunden. Das Material ist nicht zytotoxisch und weist keine akute systemische Toxizität auf.

### Beispiel 2.

### Hydrogele

PVA-Hydrogele können über Einfrier-Auftau-Prozesse hergestellt werden. 20 %ige PVA Lösungen werden in einer Petrischale bei 20 Grad eingefroren. Dieser Vorgang kann in mehreren Zyklen durchgeführt werden: 12 Stunden einfrieren und in abgedecktem Zustand wieder 2 Stunden auftauen. Die resultierenden PVA-Hydrogele sind formstabil und adhäsiv. Je höher das Molekulargewicht des PVA, desto formstabiler ist das resultierende Gel.

### Beispiel 3

### Darstellung von PVA-Schaum über Gefriertrocknung aufgeschäumter PVA-Lösungen

1 bis 30 Gew.-% PVA werden in Wasser gelöst und im Mixer aufgeschäumt. Dieser Schaum wird eingefroren und lyophilisiert. Zur Stabilisierung können ionische oder nichtionische Tenside oder auch Polymere wie PEG zugefügt werden. Die in der Lebensmittelindustrie eingesetzten Gelier- und Verdickungsmittel wie Carboxymethylcellulose können Anwendung finden.

### Beispiel 4

### Darstellung von PVA-Schaum über Gefriertrocknung von vernetzten Hydrogelen

Es können physikalisch oder chemisch vernetzte Hydrogele verwendet werden. Zur genauen Steuerung der Porengröße werden Hydrogele lyophilisiert. Die durch Gefrier-Auftau-Zyklen dargestellten oder chemisch vernetzten Hydrogele zeichnen sich durch relativ homogene Porengröße und Porenverteilung aus. Die Saugfähigkeit des Materials ist insbesondere von der Porengröße abhängig.

### Beispiel 5

### Darstellung von PVA-Schaum über Gefriertrocknung von vernetzten Partikeln

Es können physikalisch oder chemisch vernetzte Partikel verwendet werden. Die Partikelgröße kann vom Bereich Nanometer bis Millimeter variiert werden. Die Porosität wird über die Vernetzung gesteuert.

### Beispiel 6

### Darstellung von Partikeln durch Zerhacken von Patches

Es ist auch eine Herstellung von Partikeln aus festem PVA-Schaum, der nach einem der Beispiele 3 bis 5 hergestellt ist, möglich. Eine Granulation nach herkömmlichen Zerkleinerungsmethoden kann direkt nach einer Extrusionslinie nachgeschaltet sein. Die Partikelgröße ist im Bereich von Millimetern einstellbar.

### Beispiel 7

### Herstellung von PVA-Partikeln

PVA-Lösungen im Konzentrationsbereich von 2 bis 30 %, insbesondere 10 bis 20 % werden in Siliconöl im Homogenizer bei 5 rpm dispergiert.

Diese Wasser-in-Öl-Emulsionen werden Gefrier-Auftau-Zyklen unterzogen. Nach 3 Zyklen werden die Partikel mit Aceton extrahiert. Die Acetonphase wird durch eine Filtermembran von 100 nm filtriert. Die so erhaltenen PVA-Partikel werden in Vakuum getrocknet und lyophilisiert.

## Patentansprüche

1. Blutstillungsmittel umfassend mindestens einen porösen Polyvinylalkohol (PVA) in festem Zustand ausgewählt aus unvernetztem PVA mit einem Molekulargewicht von 15000 bis 400000, vernetztem PVA und Mischungen davon, wobei der poröse PVA in Form eines Pulvers mit einer Teilchengröße von 100 nm bis 1 mm vorliegt und eine Porengröße von 10 nm bis 0,05 mm aufweist.

2. Blutstillungsmittel nach Anspruch 1, **dadurch gekennzeichnet, dass** der poröse PVA in Form eines PVA-Schaums vorliegt.

3. Blutstillungsmittel nach Anspruch 2, **dadurch gekennzeichnet, dass** der Schaum offenporig ist.

4. Blutstillungsmittel nach einem der vorhergehenden Ansprüche, **dadurch gekennzeichnet, dass** es in trockenem Zustand ein Flüssigkeitsaufnahmevermögen vom 15- bis 55fachen des Produkteigengewichts aufweist.

5. Blutstillungsmittel nach einem der vorhergehenden Ansprüche, **dadurch gekennzeichnet, dass** es ein Flüssigkeitsretentionsvermögen vom 15- bis 45fachen des Produkteigengewichts aufweist.

6. Blutstillungsmittel nach einem der Ansprüche 2 bis 5, **dadurch gekennzeichnet, dass** im PVA-Schaum der Luftanteil nach Gefriertrocknung 60 bis 90 % beträgt.

7. Blutstillungsmittel nach einem der vorhergehenden Ansprüche, **dadurch gekennzeichnet, dass** dem PVA mindestens ein Wirkstoff, insbesondere mindestens eine die Blutgerinnung fördernde Substanz zugesetzt ist.

8. Blutstillungsmittel nach einem der vorhergehenden Ansprüche, **dadurch gekennzeichnet, dass** der PVA ein Molekulargewicht von 50000 bis 300000 aufweist.

9. Blutstillungsmittel nach einem der vorhergehenden Ansprüche, **dadurch gekennzeichnet, dass** der PVA aus einem Gemisch von niedermolekularen und hochmolekularen Komponenten gebildet ist, von denen mindestens eine PVA ist, wobei die hochmolekulare Komponente insbesondere hochmolekularer PVA ist.

10. Blutstillungsmittel nach einem der vorhergehenden Ansprüche, **dadurch gekennzeichnet, dass** der PVA chemisch vernetzt ist.

11. Blutstillungsmittel nach Anspruch 10, **dadurch gekennzeichnet, dass** die chemische Vernetzung durch vernetzende Veresterung vorgenommen ist.

12. Blutstillungsmittel nach einem der Ansprüche 10 oder 11, **dadurch gekennzeichnet, dass** die chemische Vernetzung mittels Vernetzern vorgenommen ist, die eine in vivo reversible Vernetzung, insbesondere eine durch chemische Hydrolyse reversible Vernetzung ergeben.

13. Blutstillungsmittel nach einem der Ansprüche 10 bis 12, **dadurch gekennzeichnet, dass** als Vernetzer mehrwertige Carbonsäuren und/oder ihre Derivate vorgesehen sind.

14. Blutstillungsmittel nach einem der Ansprüche 1 bis 9, **dadurch gekennzeichnet, dass** der PVA physikalisch vernetzt ist.

15. Blutstillungsmittel nach Anspruch 14, **dadurch gekennzeichnet, dass** die physikalische Vernetzung durch Kristallitbildung vorgenommen ist.

16. Blutstillungsmittel nach einem der vorhergehenden Ansprüche, **dadurch gekennzeichnet, dass** PVA durch über Hydroxylgruppen gebundene Reste modifiziert ist.

17. Blutstillungsmittel nach Anspruch 16, **dadurch gekennzeichnet, dass** 1 bis 10, insbesondere 1 bis 2 Reste pro PVA-Molekül vorhanden sind.

18. Blutstillungsmittel nach einem der Ansprüche 16 oder 17, **dadurch gekennzeichnet, dass** die Reste 2 bis 16 Kohlenstoffatome enthalten, insbesondere Kohlenhydrat-, Fettsäure- und/oder Alkoholreste sind.

19. Blutstillungsmittel nach einem der vorhergehenden Ansprüche, **dadurch gekennzeichnet, dass** PVA in Mischung mit einer hochmolekularen Komponente, die kein PVA ist, vorliegt.

20. Blutstillungsmittel nach Anspruch 19, **dadurch gekennzeichnet, dass** die hochmolekulare Komponente in einer Menge von 0,5 bis 4 Gew.-%, insbesondere 1 bis 2 Gew.-% vorliegt.

21. Blutstillungsmittel nach einem der Ansprüche 19 oder 20, **dadurch gekennzeichnet, dass** dem PVA als hochmolekulare Komponente ein Zuckerpolymer zugesetzt ist.

22. Blutstillungsmittel nach Anspruch 21, **dadurch gekennzeichnet, dass** das Zuckerpolymer ausgewählt ist aus der Gruppe bestehend aus Carboxymethylcellulose, Dextran, Hydroxymethylcellulose und Mischungen davon.

23. Blutstillungsmittel nach einem der vorhergehenden Ansprüche, **dadurch gekennzeichnet, dass** das Molekulargewicht des PVA so gewählt ist, dass er gegebenenfalls nach einer Hydrolyse und/oder Aufhebung der Vernetzung, im wesentlichen ohne Degradation der PVA-Moleküle über die Niere ausscheidbar ist.

24. Blutstillungsmittel nach einem der vorhergehenden Ansprüche, **dadurch gekennzeichnet, dass** seine Funktionsdauer im Behandlungsgebiet 5 bis 21 Tage, insbesondere 5 bis 14 Tage beträgt.

25. Blutstillungsmittel nach einem der vorhergehenden Ansprüche, **dadurch gekennzeichnet, dass** seine makroskopische Auflösung unter physiologischen Bedingungen, insbesondere in vivo 7, bis 60 Tage beträgt.

26. Verfahren zur Herstellung von porösem Polyvinylalkohol (PVA) in festem pulverförmigem Zustand nach einem der Ansprüche 1 bis 25 zur Verwendung als Blutstiflungsmittel in der Humanmedizin und Veterinärmedizin.

## Claims

1. Haemostatic composition comprising at least one porous polyvinyl alcohol (PVA) in the solid state selected from uncrosslinked PVA with a molecular weight of from 15 000 to 400 000, crosslinked PVA and mixtures thereof, where the porous PVA is in the form of a powder with a particle size of from 100 nm to 1 mm and has a pore size of from 10 nm to 0.05 mm.

2. Haemostatic composition according to claim 1, **characterized in that** the porous PVA is in the form of a PVA foam.

3. Haemostatic composition according to claim 2, **characterized in that** the foam is open-pore foam.

4. Haemostatic composition according to any of the preceding claims, **characterized in that** in the dry state it has a liquid uptake capacity of from 15 to 55 times the product's own weight.

5. Haemostatic composition according to any of the preceding claims, **characterized in that** it has a liquid retention capacity of from 15 to 45 times the product's own weight.

6. Haemostatic composition according to any of claims 2 to 5, **characterized in that** the air content in the PVA foam after freeze drying is 60 to 90%.

7. Haemostatic composition according to any of the preceding claims, **characterized in that** at least one active ingredient, in particular at least one substance which promotes blood clotting, is added to the PVA.

8. Haemostatic composition according to any of the preceding claims, **characterized in that** the PVA has a molecular weight of from 50 000 to 300 000.

9. Haemostatic composition according to any of the preceding claims, **characterized in that** the PVA is formed from a mixture of low molecular weight and high molecular weight components, of which at least one is PVA, where the high molecular weight component is in particular high molecular weight PVA.

10. Haemostatic composition according to any of the preceding claims, **characterized in that** the PVA is chemically crosslinked.

11. Haemostatic composition according to claim 10, **characterized in that** the chemical crosslinking is carried out by crosslinking esterification.

12. Haemostatic composition according to claim 10 or 11, **characterized in that** the chemical crosslinking is carried out by means of crosslinkers which afford a crosslinking which is reversible in vivo, in particular a crosslinking which is reversible by chemical hydrolysis.

13. Haemostatic composition according to any of claims 10 to 12, **characterized in that** polybasic carboxylic acids and/or derivatives thereof are provided as crosslinkers.

14. Haemostatic composition according to any of claims 1 to 9, **characterized in that** the PVA is physically crosslinked.

15. Haemostatic composition according to claim 14, **characterized in that** the physical crosslinking is carried out by crystallite formation.

16. Haemostatic composition according to any of the preceding claims, **characterized in that** PVA is modified by residues linked via hydroxyl groups.

17. Haemostatic composition according to claim 16, **characterized in that** 1 to 10, in particular 1 to 2, residues are present per PVA molecule.

18. Haemostatic composition according to claim 16 or 17, **characterized in that** the residues comprise 2 to 16 carbon atoms, and are in particular carbohydrate, fatty acid and/or alcohol residues.

19. Haemostatic composition according to any of the preceding claims, **characterized in that** PVA is present mixed with a high molecular weight component which is not a PVA.

20. Haemostatic composition according to claim 19, **characterized in that** the high molecular weight component is present in an amount of from 0.5 to 4% by weight, in particular 1 to 2% by weight.

21. Haemostatic composition according to claim 19 or 20, **characterized in that** a sugar polymer is added as high molecular weight component to the PVA.

22. Haemostatic composition according to claim 21, **characterized in that** the sugar polymer is selected from the group consisting of carboxymethylcellulose, dextran, hydroxymethylcellulose and mixtures thereof.

23. Haemostatic composition according to any of the preceding claims, **characterized in that** the molecular weight of the PVA is chosen such that it can be excreted via the kidney where appropriate after a hydrolysis and/or abolition of the crosslinking, substantially without degradation of the PVA molecules.

24. Haemostatic composition according to any of the preceding claims, **characterized in that** the duration of its function in the treatment area is from 5 to 21 days, in particular 5 to 14 days.

25. Haemostatic composition according to any of the preceding claims, **characterized in that** its macroscopic disintegration under physiological conditions, in particular in vivo, takes from 7 to 60 days.

26. Process for producing porous polyvinyl alcohol (PVA) in a solid state in powder form according to any of Claims 1 to 25 for use as haemostatic composition in human medicine and veterinary medicine.

## Revendications

1. Hémostatique comprenant au moins un poly(alcool vinylique) (PVA) poreux, à l'état solide, choisi parmi un PVA non réticulé ayant une masse moléculaire de 15 000 à 400 000, un PVA réticulé et des mélanges de ceux-ci, le PVA poreux se trouvant sous forme d'une poudre ayant une taille de particule de 100 nm à 1 mm et présentant une taille de pore de 10 nm à 0,05 mm.

2. Hémostatique selon la revendication 1, **caractérisé en ce que** le PVA poreux se trouve sous forme d'une mousse de PVA.

3. Hémostatique selon la revendication 2, **caractérisé en ce que** la mousse est à pores ouverts.

4. Hémostatique selon l'une quelconque des revendications précédentes, **caractérisé en ce qu'**il présente à l'état sec un pouvoir d'absorption de liquide de 15 à 55 fois le poids propre du produit.

5. Hémostatique selon l'une quelconque des revendications précédentes, **caractérisé en ce qu'**il présente un pouvoir de rétention de liquide de 15 à 45 fois le poids propre du produit.

6. Hémostatique selon l'une quelconque des revendications 2 à 5, **caractérisé en ce que** dans la mousse de PVA la proportion d'air après lyophilisation est de 60 à 90 %.

7. Hémostatique selon l'une quelconque des revendications précédentes, **caractérisé en ce que** qu'on ajoute au PVA au moins une substance active, en particulier au moins une substance favorisant la coagulation du sang.

8. Hémostatique selon l'une quelconque des revendications précédentes, **caractérisé en ce que** le PVA a une masse moléculaire de 50 000 à 300 000.

9. Hémostatique selon l'une quelconque des revendications précédentes, **caractérisé en ce que** le PVA est constitué d'un mélange de composants de faible masse moléculaire et de composants de masse moléculaire élevée, dont au moins est un PVA, le composant de masse moléculaire élevée étant en particulier un PVA de masse moléculaire élevée.

10. Hémostatique selon l'une quelconque des revendications précédentes, **caractérisé en ce que** le PVA est réticulé chimiquement.

11. Hémostatique selon la revendication 10, **caractérisé en ce que** la réticulation chimique est effectuée par estérification réticulante.

12. Hémostatique selon la revendication 10 ou 11, **caractérisé en ce** la réticulation chimique est effectuée au moyen d'agents de réticulation qui produisent une réticulation réversible in vivo, en particulier une réticulation réversible par hydrolyse chimique.

13. Hémostatique selon l'une quelconque des revendications 10 à 12, **caractérisé en ce que** des acides carboxyliques plurivalents et/ou leurs dérivés sont prévus en tant qu'agent de réticulation.

14. Hémostatique selon l'une quelconque des revendications 1 à 9, **caractérisé en ce que** le PVA est réticulé physiquement.

15. Hémostatique selon la revendication 14, **caractérisé en ce que** la réticulation physique est effectuée par formation de cristallites.

16. Hémostatique selon l'une quelconque des revendications précédentes, **caractérisé en ce que** le PVA est modifié par des radicaux liés par des groupes hydroxy.

17. Hémostatique selon la revendication 16, **caractérisé en ce que** 1 à 10, en particulier 1 ou 2 radicaux sont présents par molécule de PVA.

18. Hémostatique selon la revendication 16 ou 17, **caractérisé en ce que** les radicaux contiennent de 2 à 16 atomes de carbone, en particulier sont des restes de glucides, d'acides gras et/ou d'alcools.

19. Hémostatique selon l'une quelconque des revendications précédentes, **caractérisé en ce que** le PVA est présent en mélange avec un composant de masse moléculaire élevée qui n'est pas un PVA.

20. Hémostatique selon la revendication 19, **caractérisé en ce que** le composant de masse moléculaire élevée est présent en une quantité de 0,5 à 4 % en poids, en particulier de 1 à 2 % en poids.

21. Hémostatique selon la revendication 19 ou 20, **caractérisé en ce qu'**un polymère glucidique est ajouté en tant que composant de masse moléculaire élevée au PVA.

22. Hémostatique selon la revendication 21, **caractérisé en ce que** le polymère glucidique est choisi dans le groupe constitué par la carboxyméthylcellulose, le dextrane, l'hydroxyméthylcellulose et des mélanges de ceux-ci.

23. Hémostatique selon l'une quelconque des revendications précédentes, **caractérisé en ce que** la masse moléculaire du PVA est choisie de manière qu'il puisse être éliminé par les reins, éventuellement après une hydrolyse et/ou une suppression de la réticulation, pratiquement sans dégradation des molécules de PVA.

24. Hémostatique selon l'une quelconque des revendications précédentes, **caractérisé en ce que** sa durée de fonctionnement dans le domaine de traitement va de 5 à 21 jours, en particulier de 5 à 14 jours.

25. Hémostatique selon l'une quelconque des revendications précédentes, **caractérisé en ce que** sa dissolution macroscopique dans des conditions physiologiques, en particulier in vivo, est de 7 à 60 jours.

26. Procédé pour la préparation de poly(alcool vinylique) (PVA) poreux, à l'état solide pulvérulent, selon l'une quelconque des revendications 1 à 25, pour l'utilisation en tant qu'hémostatique en médecine humaine et en médecine vétérinaire.
